(19) Europäisches Patentamt
European Patent Office
Office européen des brevets

(11) Publication number: **0 479 380 A1**

(12) **EUROPEAN PATENT APPLICATION**

(21) Application number: **91202514.5**

(51) Int. Cl.[5]: **C12Q 1/48**

(22) Date of filing: **27.09.91**

(30) Priority: **05.10.90 US 594800**

(43) Date of publication of application:
**08.04.92 Bulletin 92/15**

(84) Designated Contracting States:
**AT BE CH DE ES FR GB GR IT LI LU NL SE**

(71) Applicant: **EASTMAN KODAK COMPANY**
**343 State Street**
**Rochester, New York 14650-2201(US)**

(72) Inventor: **Nealon, Daniel Anthony, c/o EASTMAN KODAK COMP.**
**Patent Department, 343 State Street**
**Rochester, New York 14650-2201(US)**
Inventor: **Daggett, Stephen Gregory, c/o EASTMAN KODAK COMP.**
**Patent Department, 343 State Street**
**Rochester, New York 14650-2201(US)**
Inventor: **Norton, Gary Edward, c/o EASTMAN KODAK COMP.**
**Patent Department, 343 State Street**
**Rochester, New York 14650-2201(US)**

(74) Representative: **Nunney, Ronald Frederick Adolphe et al**
**Kodak Limited Patent Department Headstone Drive**
**Harrow Middlesex HA1 4TY(GB)**

(54) **Enzymatic assay for carbon dioxide.**

(57) A method for the assay of carbon dioxide in body fluids is disclosed. The method involves:
a) mixing, at a pH in the range of 6.0 to 8.5, a measured amount of said body fluid with excess amounts of phosphoenolpyruvate; malate dehydrogenase; from 0.2 to 1.5 mmol/L of reduced nicotinamide adenine dinucleotide; from 0.5 to 300 U/L phosphoenolpyruvate carboxykinase; from 0.1 to 10.0 mmol/L of each of the cofactors magnesium and manganese ions; and from 0.1 to 10 mmol/L of a sulfhydryl agent and
b) spectrophotometrically measuring the change in the concentration of the reduced form of nicotinamide adenine dinucleotide in the mixture at a substantially constant temperature and pH in the range of 6.0 to 8.5.

EP 0 479 380 A1

The present invention relates to clinical chemistry. In particular, it relates to a method for the quantitative assay of carbon dioxide in body fluids, such as blood serum.

Significant amounts of carbon dioxide are generated by humans. In general, only a small portion of the $CO_2$ generated is utilized. The rest is considered waste material and is generally eliminated through body fluids, such as the blood stream. As a waste product, the concentration of carbon dioxide in the blood stream has a profound effect on body functions. Thus, the measurement of carbon dioxide content in the blood stream or other body fluids is an important tool for precise and accurate medical diagnosis.

The term carbon dioxide content, as applied to body fluids, means the sum of bicarbonate ions, carbonic acid and dissolved carbon dioxide according to the equilibrium:

$$CO_2 + H_2O \rightleftarrows H_2CO_3 \rightleftarrows H^+ + HCO_3^-$$

U.S. Patent 3,974,037 discloses several enzymatic reaction schemes for the determination of carbon dioxide. These disclosed reactions are (1), (2), (3) and (4) below. Each generates, as one of the products, oxalacetate which can be measured with reaction (5).

$$(1)\ \text{phosphoenolpyruvate} + HCO_3^- \xrightarrow{\text{PEP carboxylase}} \text{oxalacetate} + P_i;$$

where PEP carboxylase is phosphoenolpyruvate carboxylase and Pi is inorganic phosphorus;

$$(2)\ \text{ATP} + \text{pyruvate} + HCO_3^- \xrightarrow[\text{carboxylase}]{\text{pyruvate}} \text{oxalacetate} + \text{ADP} + P_i;$$

$$(3)\ \text{phosphoenolpyruvate} + HCO_3^- + \text{ADP} \xrightarrow[\text{carboxykinase}]{\text{PEP}} \text{oxalacetate} + \text{ATP};$$

where ADP stands for adenosine diphosphate and ATP stands for adenosine triphosphate. In the reaction defined by (3), the ADP can be replaced by other nucleotide diphosphates depending on the source from which PEP carboxykinase is prepared.

$$(4)\ \text{phosphoeolpyruvate} + HCO_3^- + P_i \xrightarrow[\text{carboxykinase (pyrophosphate)}]{\text{PEP}} \text{oxalacetate} + PP_i;$$

where $PP_i$ stands for inorganic pyrophosphate; and

$$(5)\ \text{oxalacetate} + \text{NADH} + H^+ \xrightarrow{\text{MDH}} \text{malate} + \text{NAD}^+$$

where MDH is malate dehydrogenase, NADH is the reduced form of nicotinamide adenine dinucleotide and $NAD^+$ is the oxidized form.

Our attempts to measure carbon dioxide in body fluids using reactions (3) and (5) were unsuccessful. The results we obtained using only the reagents included in the reaction scheme gave assay results which did not respond to quantitative changes in carbon dioxide concentration; that is, the assay was analytically insensitive.

The present invention overcomes the above stated problem. The method of the present invention for assaying carbon dioxide in aqueous body fluids comprises the steps of:

a) mixing, at a pH in the range of 6.0 to 8.5, a measured amount of said body fluid with excess amounts of phosphoenolpyruvate; malate dehydrogenase; from 0.2 to 1.5 mmol/L of reduced nicotinamide adenine dinucleotide; from 50 to 300 U/L phosphoenolpyruvate carboxykinase; from 0.1 to 10.0 mmol/L of each of the cofactors magnesium and manganese ions; and from 0.1 to 10 mmol/L of a sulfhydryl agent and

b) spectrophotometrically measuring the change in the concentration of the reduced form of nicotinamide adenine dinucleotide in the mixture at a substantially constant temperature and pH in the range of 6.0 to 8.5.

The method of assay in the above method is enzymatic, as specified by reaction (3).

In the absence of, or the presence of an excess of any one of magnesium ions, manganese ions and a sulfhydryl agent (for example, N-Acetyl-L-cysteine, diethioerythritol, dithiothreitol), PEP carboxykinase is not active and, therefore the assay response to carbon dioxide is not sensitive enough to determine carbon dioxide quantitatively. By excess we mean amounts of each reagent above the amounts specified hereinafter.

At NADH levels below 0.2. mmol/L, the assay cannot support the dynamic range required to provide clinically useful carbon dioxide results. Concentrations of 0.2 to 1.5 mmol/L are useful. By dynamic range we mean the range of carbon dioxide levels, typically 5-45 mmol/L, that are clinically useful to ensure proper diagnosis.

The pH must be in the range of 6.0 to 8.5. Below pH 6.0, the PEP carboxykinase is not stable and above pH 8.5, the level of carbon dioxide is too low for the assay to be analytically sensitive.

The method of the invention only works within a very narrow level of PEP carboxykinase activity. These activity levels are 50 to 300 U/L as defined hereinafter in example 1. Use of activity levels above or below these amounts results in an assay which cannot accurately and reliably distinguish carbon dioxide levels.

The presence of both magnesium ($Mg^{++}$) and manganese ($Mn^{++}$) ions as metal ion cofactors for PEP carboxykinase must be in a concentration of 0.1 to 10.0 mmol/L for the method to work.

The solution assay can be carried out by mixing each reagent in the amounts required by the method of the invention with a measured amount of body fluid, such as human blood.

The amount of reagents used in a particular assay are easily determined by those skilled in analytical chemistry, based on the stoichiometric relations of equations (3) and (5) coupled with the ranges of $CO_2$ normally seen in the body fluid samples being tested. Amounts of total carbon dioxide outside of such ranges can be dealt with by making adjustments based on stoichiometric relations and other adjustments, such as dilution or concentrating a sample, known to analytical chemists.

The change in the concentration of NADH, according to reaction (5), is directly proportional to the decrease in absorbance of light between 290 and 380 nm, preferably 340 nm, at a constant temperature between 25°C and 45°C and a constant pH, usually between 6.0 and 8.5. NADH concentration can be measured readily by those skilled in the art using conventional spectrophotometric procedures. The change in the concentration of NADH is proportional to the amount of oxalacetate formed. The formation of oxalacetate is a function of the concentration of carbon dioxide in the system. Thus the change in absorbance at 340 nm can be used as a direct measure of the original concentration of carbon dioxide in the sample fluid. Other wavelengths, for example 366 nm, can also be used for the foregoing purpose.

All of the reagents used in carrying out the necessary enzymatic reactions are available commercially.

## Example 1

A kinetic assay for carbon dioxide can be carried out with the following reagent mixture at the final concentrations indicated:

| | | Ranges |
|---|---|---|
| Phosphoenolpyruvate | 4.4 mmol/L | > 2 mmol/L |
| $MgCl_2$ | 4.4 mmol/L | 0.1-10 mmol/L |
| $MnCl_2$ | 0.2 mmol/L | 0.1-10 mmol/L |
| N-acetyl-L-cysteine | 7.3 mmol/L | 0.1-10 mmol/L |
| NADH | 0.3 mmol/L | 0.2-1.5 mmol/L |
| Guanosine 5'-diphosphate | 1.6 mmol/L | 0.5-20 mmol/L |
| Malate dehydrogenase | 140 U | > 10 U |
| PEP carboxykinase | 2.5 U | 0.5-3.0 U |
| Tricine | 150 mmol/L | 10-500 mmol/L |

The mixture is adjusted to a total volume of 10 mL and a pH of 7.5 (pH range 6.0-8.5). One unit of PEP carboxykinase is defined as that amount of the latter enzyme which catalyzes the carboxylation of 0.001 mmol of phosphoenolpyruvate/minute under the assay conditions. The source of the phosphoenolpyruvate carboxykinase in this example is rabbit liver.

Using this assay mixture, a standard curve was generated with carbon dioxide standards over the range of 5 mmol/L to 60 mmol/L. The carbon dioxide sample size was 20 µL in a total assay volume of 240 µL. The change in absorbance per minute (ΔAbs/min) in milliabsorbance units/minute was measured for each standard. The results are given in Table 1.

TABLE 1

| Carbon Dioxide (mmol/L) | Δm Abs/min. |
|---|---|
| 5 | 115 |
| 10 | 172 |
| 20 | 286 |
| 30 | 395 |
| 40 | 492 |
| 60 | 674 |

A curve prepared from these absorbance values showed good linearity over the range of concentration tested.

Example 2

Using the same procedure and reagents set forth in Example 1, carbon dioxide concentration was measured in commercially available human serum-based control products. Carbon dioxide values for these controls were assigned by a thermal conductivity based reference method according to Van Slyke and others, J. Biol. Chem., 61:523, 1924. The reference procedure was carried out on a Corning 965 carbon dioxide analyzer. The manufacturer's reported reference values for these control products were 13 and 25 mmol/L. The results are shown in Table 2. Using the equation of a straight line, $y = mx + b$, the amount of carbon dioxide present in the human serum-based control products can be calculated from the slope and the intercept of a standard curve generated with the data shown in Table 1.

Table 2

| Carbon Dioxide Reference Value (mmol/L) | Δm Abs/min. | Carbon Dioxide Calculated Value (mmol/L) |
|---|---|---|
| 13 | 210 | 13.2 |
| 25 | 336 | 25.6 |

The same straight line can be used to calculate the carbon dioxide levels in aqueous carbon dioxide solutions (that is, those of Example 1). This data is shown in Table 3.

Table 3

| Carbon Dioxide Reference Value (mmol/L) | Carbon Dioxide Calculated Value (mmol/L) |
|---|---|
| 5 | 5.4 |
| 10 | 9.3 |
| 20 | 19.4 |
| 30 | 30.4 |
| 40 | 39.2 |
| 60 | 58.5 |

The values of ΔAbs/min. shown in Tables 1 and 2 are mean values for several replications. In order to assess the precision of the determination, the standard deviation for the values shown in Tables 1 and 2 were calculated and converted to mmol/L values. The reference values were compared with the mean values and standard deviation for each measurement. These reference values were also compared with measurements made on other commercial devices using data from Sterling, R.E., Flores, O.R., Clinical Chemistry, 18:544, 1972, and U.S. Patent 3,974,037. Sterling, R.E., Flores, O.R., Clinical Chemistry, 18:544, 1972, and U.S. Patent 3,974,037.

These comparisons showed that the method of the present invention for measuring carbon dioxide achieved at least equivalent or better precision than many other currently existing methods for measuring carbon dioxide.

**Claims**

**1.** A method for the assay of carbon dioxide in body fluids comprising the steps of:
a) mixing, at a pH in the range of 6.0 to 8.5, a measured amount of said body fluid with excess amounts of phosphoenolpyruvate; malate dehydrogenase; from 0.2 to 1.5 mmol/L of reduced nicotinamide adenine dinucleotide; from 50 to 300 U/L phosphoenolpyruvate carboxykinase; from 0.1 to 10.0 mmol/L of each of the cofactors magnesium and manganese ions; and from 0.1 to 10 mmol/L of a sulfhydryl agent and
b) spectrophotometrically measuring the change in the concentration of the reduced form of nicotinamide adenine dinucleotide in the mixture at a substantially constant temperature and pH in the range of 6.0 to 8.5.

**2.** The method of Claim 1, wherein step a) is carried out at a pH of 7.5.

**3.** The method of Claim 1 or 2, wherein the sulfhydryl agent is selected from the group consisting of N-acetyl-L-cysteine, dithioerythritol or dithiothreitol.

**4.** The method of Claim 2, wherein the sulfhydryl agent is N-acetyl-L-cysteine.

**5.** The method of Claim 1, wherein the change in concentration of the reduced form of nicotinamide adenine dinucleotide is determined spectrophotometrically by measuring the absorbance at a wavelength of 290 to 380 nm at a constant temperature of between 25 and 45 °C and a constant pH of between 6.0 and 8.5.

**6.** The method of Claim 5, wherein measurement is carried out at 340 nm.

**7.** The method of claim 1 wherein the pH is 7.5, and the mixture of step a) contains 0.3 mmol/L of reduced nicotinamide adenine dinucleotide; 250 U/L of phosphoenolpyruvate carboxykinase; 4.4 mmol/L of magnesium ions and 0.2 mmol/L manganese ions; and 7.3 mmol/L of N-acetyl-L-cysteine.

European Patent Office

# EUROPEAN SEARCH REPORT

Application Number

**EP 91 20 2514**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-3 974 037 (THOMAS H. ADAMS)<br>* the whole document * *<br>– – – | 1-7 | C 12 Q 1/48 |
| A | DE-A-2 409 886 (MEDI-COMPUTER CORP.)<br>* the whole document * *<br>– – – | 1-7 | |
| A | BIOLOGICAL ABSTRACTS, vol.62, no.6, 15.09.1976 PHILA-DELPHIA,PA,US; abstract no. 31043 BENTLE,L.A. et al. :"Interaction of Anions and Divalent Metal Ions with Phosphoenolpyruvate & J. BIOL. CHEM. 251(10), 2916-2921(1976)<br>– – – – – | 1-7 | |

| TECHNICAL FIELDS SEARCHED (Int. Cl.5) |
|---|
| C 12 Q |

The present search report has been drawn up for all claims

| Place of search | Date of completion of search | Examiner |
|---|---|---|
| The Hague | 03 December 91 | DOEPFER K-P. |

CATEGORY OF CITED DOCUMENTS

- X : particularly relevant if taken alone
- Y : particularly relevant if combined with another document of the same catagory
- A : technological background
- O : non-written disclosure
- P : intermediate document
- T : theory or principle underlying the invention
- E : earlier patent document, but published on, or after the filing date
- D : document cited in the application
- L : document cited for other reasons
- & : member of the same patent family, corresponding document